# EUROPEAN PATENT APPLICATION

(11) **EP 4 445 905 A1**
(43) Date of publication of application: **16.10.2024**
(21) Application number: 23382344.2
(22) Date of filing: 14.04.2023
(51) Int. Cl.: A61K 35/16, A61P 19/00, A61P 21/00, A61P 37/06, C12N 5/078

(54) **PLASMA ENRICHED IN PLATELETS AND PLASMA MOLECULES**

(71) Applicant: Santxa Research S.L.U., 01008 Vitoria-Gasteiz (ES)
(72) Inventor: SÁNCHEZ ÁLVAREZ, Mikel, E-01008 Vitoria-Gasteiz (ES); SÁNCHEZ ARIZMENDIARRIETA, Pello, E-01008 Vitoria-Gasteiz (ES); DELGADO SAN VICENTE, Diego, E-01008 Vitoria-Gasteiz (ES); BEITIA SAN VICENTE, Maider, E-01008 Vitoria-Gasteiz (ES)
(74) Representative: ABG Intellectual Property Law, S.L.

(57) **Abstract**

The present invention belongs to the field of biotechnology and concerns a method of obtaining a plasma enriched in platelets and plasma molecules and the plasma obtained by this method. The method comprises the concentration of a red blood cell-free plasma with a hydroxyalkyl acrylic hydrogel.

## Description

### FIELD OF THE INVENTION

The present invention belongs to the field of biotechnology and concerns a method of obtaining a plasma enriched in platelets and plasma molecules and the plasma obtained by this method.

### BACKGROUND ART

In recent years, advanced biological therapies and regenerative medicine have become a promising alternative to conventional treatments. The use of Platelet Rich Plasma (PRP) is one of the technologies that has been most widespread and consolidated in several fields of medicine.

PRP is a pool of blood products in which platelets are found in higher concentrations than in blood. Once PRP is activated, plasma fibrinogen polymerises into a three-dimensional transient fibrin scaffold, trapping various growth factors, microparticles and other biomolecules released by platelet degranulation and present in plasma. The growth factors and biomolecules sequestered in the fibrin scaffold are released in a gradual and sustained manner as fibrinolysis of the scaffold occurs, making PRP well suited to enhance and accelerate the natural process of tissue repair and ultimately reduce recovery times.

In particular, released growth factors as well as plasma molecules trigger biological processes aimed at repairing damaged tissue, e.g. angiogenesis, chemotaxis, cell migration or proliferation via cell membrane signalling.

It is therefore evident that plasma is a cocktail of molecules of a very diverse nature and biological role. In fact, not all molecules in plasma are beneficial to all clinical contexts. It is a difficult task to modulate the balance of the different PRP biomolecules (anabolic, catabolic, platelet, plasmatic, etc.) to favour certain biological processes and hence particularly suit a specific clinical context.

Clinical practices where PRP-based therapies have generally burst into are the field of orthopaedics and sports medicine. This is evidenced by the growing number of studies related to pathologies such as osteoarthritis, tendinopathies or ligament injuries.

Some types of growth factors circulate in plasma, e.g. insulin-like growth factor (IGF), which promotes wound healing, bone formation, striated muscle myogenesis and keratinocyte migration, and has been proven particularly useful in musculoskeletal regenerative therapy, such as in articular cartilage repair, or against osteoporosis, age-related wasting, muscular dystrophy or osteoarthritis among others (Leon A Bach, Clin Biochem Rev Vol 25 155-160 August 2004); Hepatocyte growth factor (HGF), which promotes dynamic biological responses that support morphogenesis (e.g., epithelial tubulogenesis), regeneration, and the survival of cells and tissues, and has been proven particularly useful in musculoskeletal regenerative therapy, such as against articular cartilage injury, skeletal muscle injury, ligament injury or rheumatoid arthritis among others (Matsumoto et al., Biomedicines 2014, 2, 275-300).

As a result, a large number of PRP products have appeared on the market and, although these products are generally labelled as PRP, they have different properties, such as varying concentrations of platelets, plasma molecules, or the presence or absence of leukocytes, and are each differently suited to different clinical contexts. The device and method employed in the preparation of PRP has an important impact on these properties. Examples of commercially available systems for preparing PRP are Magellan (Arteriocyte Medtronic), ACP or Angel (Arthrex), PRGF-Endoret (BTI Biotechnology Institute), MTF (Cascade), Secquire (Pure PRP Emcyte), RegenKit (RegenLab), GPS (Zimmer Biomet), Ortho Pras (Proteal).

Despite the great expansion of PRP in recent years and the large number of PRP systems on the market, all of them are based on the principle of centrifugation to separate the blood fractions.

In addition to centrifugation, new methods of obtaining PRP are being developed to improve certain aspects of centrifugation such as processing times or dependence on the skill of the technical staff. Thus, other techniques are based on filtration by which platelets are separated from the rest of the blood components, increasing the speed of the process, but altering the composition of the PRP due to the inclusion of aqueous dilutions.

Filtration has also been combined with sedimentation, whereby the plasma fraction is obtained first and then the filtration process is carried out to obtain platelets, achieving a concentration of biomolecules and growth factors. However, the timescales of this methodology are excessive and the inclusion of aqueous solutions in the process complicates its clinical application.

In recent years, new modifications have been introduced in the formulation of PRP, even varying the concentration of certain external proteins to those secreted by platelets after activation. However, achieving a desired improved biological activity is not as simple as externally adding proteins, as said biological activity usually depends on a complex suite of biomolecular interactions.

Another alternative for dehydration of PRP, and thus concentration of both cellular and molecular components, is the use of water-absorbing biomaterials such as polyacrylamide hydrogels. Methods using hydrogels to concentrate platelets and molecules have been described, such as the method described in patent document WO2006086199A1.

However, there is an ongoing need not only for the development of new methodologies that are capable of producing PRP with a higher capacity for platelet and protein concentration, but also for the production of PRPs which naturally (i.e. not externally added) possess high levels of particular beneficial molecules such that the resulting product possesses greater bioactivity on proliferation in cell cultures and are thus well-suited to be used in particular clinical contexts.

### SUMMARY OF THE INVENTION

This invention shows the development of a new methodology for the production of an all-natural plasma enriched in platelets and plasma molecules. This allows for a high level of biosafety and takes advantage of the biological properties of the biomolecules present in plasma.

The authors of the present invention have shown that the plasma of the invention is superior to conventional plasmas in the proportion of total and specific plasma proteins present and in its ability to induce dermal fibroblast cell proliferation. In particular, the plasma obtained by the developed method contains, surprisingly, a higher concentration of the growth factors IGF-I and HGF, than other plasmas in the state of the art.

The new method is also very advantageous because it requires minimal preparation for use by the doctor.

Thus, in a first aspect, the present invention relates to an *in vitro* method, hereinafter the method of the invention, for producing a plasma enriched in platelets and plasma molecules comprising:
(i) obtaining a red blood cell-free plasma from an anticoagulated blood sample of a subject, wherein the plasma comprises platelets and plasma molecules; and
(ii) concentrating the platelets and plasma molecules comprised in the plasma obtained in step i) by contacting said plasma with a hydrogel, wherein the hydrogel comprises a polymer network, and the polymer network comprises monofunctionalized N-hydroxyalkyl acrylamide monomeric units; or N-hydroxyalkyl alkylacrylamide monomeric units; or hydroxyalkyl acrylate monomeric units; or hydroxyalkyl alkylacrylate monomeric units; and is cross-linked.

In a second aspect, the present invention relates to a plasma enriched in platelets and plasma molecules obtained by the method of the invention.

In a third aspect, the invention relates to a plasma enriched in platelets and plasma molecules according to the second aspect of the invention for use in medicine.

In an embodiment, the present invention relates to a plasma enriched in platelets and plasma molecules according to the second aspect of the invention for use in regenerative medicine, preferably in musculoskeletal regenerative therapy.

In an embodiment, the invention relates to the plasma enriched in platelets and plasma molecules according to the second aspect of the invention for use in the treatment of inflammatory diseases.

In an embodiment, the invention relates to the plasma enriched in platelets and plasma molecules according to the second aspect of the invention for use in the treatment of rheumatic diseases.

In another aspect, the invention relates to the cosmetic use of plasma enriched in platelets and plasma molecules of the invention.

In a final aspect, the invention relates to the *in vitro* use of a hydrogel as defined in the first aspect of the invention for concentrating blood or plasma.

### DESCRIPTION OF THE FIGURES

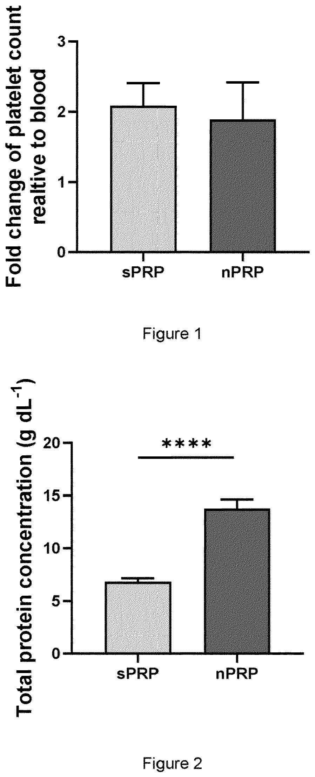

**Figure 1****.** Relative platelet levels in the nPRP and sPRP formulations. Plasmas obtained from the different systems were analysed to determine their platelet content, which was relative to baseline blood levels. Statistical significance between the two PRPs was calculated by t-test.
**Figure 2****.** Relative total protein levels of sPRP and nPRP relative to blood. Plasmas obtained from the different systems were analysed for protein content, which were relative to baseline blood levels. Statistical significance for total protein concentration between nPRP and sPRP was calculated by t-test (**** p<0.0001).

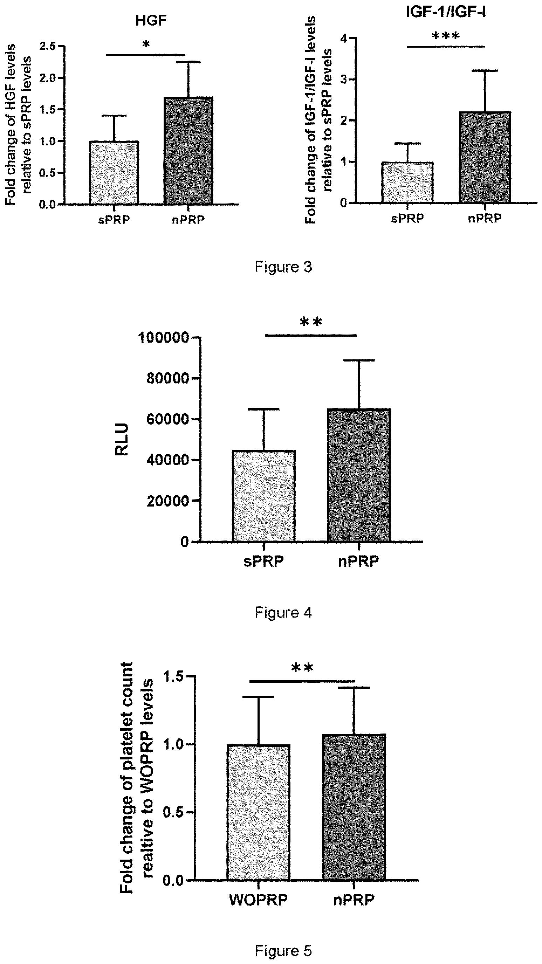

**Figure 3****.** Relative levels of plasma growth factors in the sPRP and nPRP formulations relative to sPRP (fold change of sPRP=1). Plasmas obtained from the different systems were analysed for HGF and IGF-1 content. Statistical significance between sPRP and nPRP was calculated by paired t-test (* p<0.05; *** p<0.001).
**Figure 4****.** Average viability levels of dermal fibroblasts cultured with the different sera. Cells were cultured with media supplemented with the sera from the different plasma concentrates and viability was measured at 96h. Statistical significance between sPRP and nPRP was calculated by ANOVA for normally distributed data and Kruskal-Wallis for parametrically distributed data (** p<0.01).
**Figure 5****.** Relative platelet levels for nPRP formulations relative to WOPRP levels. The statistical significance between the two PRPs was calculated using t-test (**p<0.01).

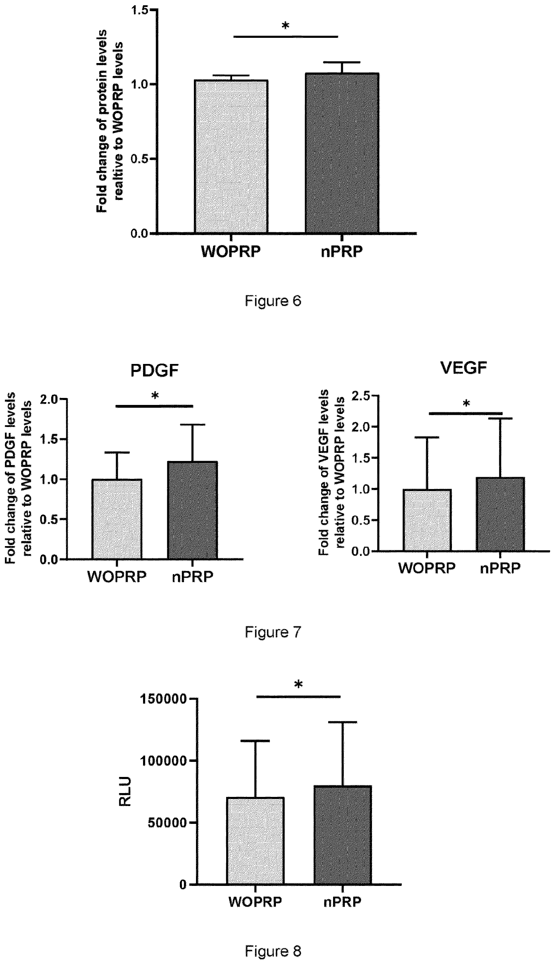

**Figure 6****.** Relative levels of total proteins for nPRP relative to WOPRP levels. The statistical significance for the concentration of total proteins between nPRP and WOPRP was calculated by t-test (*p<0.05).
**Figure 7****.** Relative levels of platelet growth factors in WOPRP and nPRP formulations relative to WOPRP (fold change of WOPRP=1). The plasmas obtained through the different systems were analyzed to determine their content in PDGF and VEGF. The statistical significance between WOPRP and nPRP was calculated by paired t-test (*p<0.05).
**Figure 8****.** Viability levels of dermal fibroblasts. The cells were cultured with media supplemented with sera from a donor processed according either to the prior art (WOPRP) or to the present invention (nPRP), and viability was measured at 96h.The experiment was repeated for 9 different donors and the average is shown. The statistical significance between WOPRP and nPRP was calculated using t-test (* p<0.05).

### DETAILED DESCRIPTION OF THE INVENTION

All terms as used in the present application, unless otherwise indicated, are to be understood in their common meaning as known in the art. Other more specific definitions for certain terms as used in the present application will be defined later in this section and are intended to apply uniformly throughout the specification and claims, unless a definition expressly set forth otherwise provides a broader definition.

### Method of the invention

In a first aspect, the invention relates to an *in vitro* method, hereinafter the method of the invention, for producing a plasma enriched in platelets and plasma molecules comprising:
(i) obtaining a red blood cell-free plasma (i.e. a plasma free of red blood cells) from an anticoagulated blood sample of a subject, wherein the plasma comprises platelets and plasma molecules; and
(ii) concentrating the platelets and plasma molecules comprised in the plasma obtained in step i) by contacting said plasma with a hydrogel, wherein the hydrogel comprises a polymer network, and the polymer network comprises monofunctionalized N-hydroxyalkyl acrylamide monomeric units; or N-hydroxyalkyl alkylacrylamide monomeric units; or hydroxyalkyl acrylate monomeric units; or hydroxyalkyl alkylacrylate monomeric units; and is cross-linked.

In the present invention the term "*in vitro*" means that the method is not carried out in the body of a human or animal subject, but in cells or fluids isolated from that subject, such as in a test tube.

The term "subject" as used herein, refers to an animal, such as a human, a nonhuman primate (e.g., chimpanzees and other apes and monkey species); farm animals, such as birds, fish, cattle, sheep, pigs, goats and horses; domestic mammals, such as dogs and cats; laboratory animals including rodents, such as mice, rats and guinea pigs. The term subject refers preferably to a mammal, more preferably a primate, and even more preferably, a human, male or female, of any age or race. In addition, the subject can be a healthy subject or a subject diagnosed with any pathology or disease.

In the present invention, the term 'plasma' or the expression 'blood plasma', refers to the non-cellular fluid portion of the blood of humans or animals prior to coagulation. It is composed of 90 % water, 7 % protein and the remaining 3 % fat, glucose, vitamins, hormones, oxygen, carbon dioxide and nitrogen, as well as metabolic waste products such as uric acid. It is the major component of blood, accounting for approximately 55% of the total blood volume, while the remaining 45% is made up of formed elements. The viscosity of blood plasma is 1.5 times that of water. The main plasma proteins are: albumin, which is also involved in lipid transport; globulins, which are mainly related to the body's defence mechanisms (e.g. immunoglobulins) or to iron transport (transferrin); fibrinogen, a protein that is essential for blood clotting; prothrombin, a protein involved in the clotting process; and low- and high-density lipoproteins (LDL and HDL, respectively) involved in cholesterol transport.

The set of plasma proteins, also called "plasma factors", performs the functions of, among others:
- maintaining plasma volume and blood volume,
- protecting and maintaining the pH stability of the blood,
- participating in blood viscosity, thereby contributing minimally to peripheral vascular resistance and vascular pressure (blood pressure), and
- intervening in the electrochemical equilibrium ion concentration (so-called Donnan effect).

The term "platelets", as used herein, refers to small (2-3 micrometres in diameter), oval, nucleusless units of cells generated under normal conditions in the bone marrow from the cytoplasmic fragmentation of megakaryocytes. Platelets circulate in the blood of all mammals and are involved in haemostasis, participating in the formation of blood clots. Platelets release a large number of growth factors and/or cytokines, including platelet-derived growth factor (PDGF, also abbreviated as PDGFAA), a potent chemotactic agent, and transforming growth factor beta (TGF-β1), which stimulates the formation of the extracellular matrix. Depending on certain conditions, platelets also synthesise many other molecules, as discussed above, essential to the inflammatory process, and to the repair and regenerative process of tissues. These growth factors or molecules have been shown to play an important role in connective tissue regeneration. Other proteins and growth factors produced by platelets and associated inflammatory, reparative and regenerative processes include basic fibroblast growth factor (BFGF), insulin-like growth factor 1 (IGF-1), insulin-like growth factor 2 (IGF-2), epithelial growth factor (EGF), hepatocyte growth factor (HGF), vascular endothelial growth factor (VEGF), interleukin-8, keratinocyte growth factor, connective tissue growth factor. The local application of all these growth factors, molecules and proteins produced by platelets participates in and accelerates the healing process of different lesions. Platelets also release large amounts of thrombin and adenosine diphosphate ADP, calcium, thromboxane A2, PF4, PAI-1, fibrinogen, fibronectin, thrombomodulin, FV, FXIII, RANTES, thrombospondin, WWF PF-3, serotonin and hydrolytic enzymes, among others.

In the present invention, the term 'plasma molecules', 'plasma biomolecules' or 'non-platelet biomolecules' refers to biomolecules contained in blood plasma, which may be not stored or not stored exclusively in platelets.

Preferably, "plasma molecules" refers to growth factors. However, the term "plasma molecules" can also include ions and proteins other than growth factors. Preferably, the term "plasma molecules" refers to biomolecules that cannot be concentrated by centrifugation techniques without damaging the platelets and/or the biomolecules themselves after said centrifugation. Concentration refers to an increase in the concentration of the total proteins as defined hereinbelow.

The term "plasma protein" as used herein refers to any protein contained in blood plasma. Blood plasma proteins comprise albumin, alpha/macroglobulin, antichymotrypsin, antithrombin, antitrypsin, Apo A, Apo B, Apo C, Apo D, Apo E, Apo F, Apo G, Apo Xlla, C 1 -inhibitor, C-reactive protein, C7, C1r, C1s, C2 C3, C4, C4bP, C5, C6, C1q, C8, C9, carboxypeptidase N, ceruloplasmin, factor B, factor D, factor H, factor I, factor IX, factor V, factor VII, factor Vila, factor VIII, factor X, factor XI, factor XII, factor XIII, fibrinogen, fibronectin, haptoglobin, haemopexin, heparin cofactor II, histidine rich GP, IgA, IgD, IgE, IgG, ITI, IgM, quininase II, quininogen HPM, lysozyme, PAI 2, PAI I, PCI, plasmin, plasmin inhibitor, plasminogen, prealbumin, prekallikrein, properdin, protease nexin, protein C, protein S, protein Z, prothrombin, serum amyloid protein (SAP), TFPI, thiol proteinase, thrombomodulin, tissue factor (TF), TPA, transcolabamine II, transcortin, transferrin, vitronectin, and Willebrand factor. Blood plasma proteins also include some hormones, including the insulin-like growth factors, e.g. IGF-I.

In particular, plasma proteins comprise the coagulation proteins, i.e. the plasma proteins involved in the chain of cascade reactions leading to the formation of a blood clot. Coagulation proteins include factor I (fibrinogen), factor II (prothrombin), factor V (proaccelerin), factor VII (proconvertin), factor VIII (anti-hemophilic factor A), factor IX (anti-hemophilic factor B), factor X (Stuart factor), factor XI (Rosenthal factor or PTA), factor XII (Hageman factor), factor XIII (fibrin stabilising factor or FSF), PK (prekallikrein), KHPM (high molecular weight kininogen), factor III (thromboplastin or tissue factor), heparin cofactor II (HCII), protein C (PC), thrombomodulin (TM), protein S (PS), Willebrand factor (Wf) and tissue factor pathway inhibitor (TFPI), or tissue factors.

In some embodiments, the plasma protein consists of an enzymatically active coagulation protein. Enzymatically active coagulation proteins include activated forms of factor II (prothrombin), factor VII (proconvertin), factor IX (anti-hemophilic factor B), factor X (factor Stuart), factor XI (Rosenthal factor or PTA), factor XII (Hageman factor), factor XIII (fibrin stabilising factor or FSF) and PK (Prekallikrein).

As used herein, "plasma enriched in plasma molecules" refers to plasma that has undergone a process that increases its concentration of non-platelet biomolecules. In a preferred embodiment, it refers to plasma that has undergone a process that increases the concentration of its total proteins as described hereinbelow.

The term "blood", as used herein, refers to a tissue fluid that flows through the capillaries, veins and arteries of all vertebrates. Its characteristic red colour is due to the presence of the red pigment content of erythrocytes. It is a specialised type of connective tissue with a liquid colloidal matrix and a complex constitution. In fact, blood comprises a solid phase made up of cells (including leukocytes, erythrocytes and platelets) and a liquid phase, represented by plasma. Its main function is logistic distribution and systemic integration, whose containment in vessels (vascular space) supports its distribution (blood circulation) throughout most of the body.

The term 'erythrocytes' or 'red blood cells' or 'RBCs' or 'red cells' as used in the present invention refers to the blood corpuscles which will transport oxygen and carbon dioxide in the blood. Corpuscles are known to lack nuclei and organelles (only in mammals), so they cannot strictly be considered as cells. They contain some enzymatic pathways, and their cytoplasm is almost entirely occupied by haemoglobin, a protein responsible for oxygen transport. The plasma membrane of erythrocytes contains the glycoproteins that define the different blood groups and other cell identifiers. Erythrocytes are disc-shaped, biconcave, depressed in the centre; this increases the effective surface area of the membrane. Mature RBCs lack a nucleus, because it is shed in the last stage of maturation in the bone marrow before entering the bloodstream.

The term "anticoagulated" is used herein to refer to blood that has been treated with an anticoagulant. The term "anticoagulant" as used in the present invention refers to an endogenous or exogenous substance that interferes with or inhibits blood clotting. Non-limiting examples of anticoagulants are antithrombin III activators and heparin, low molecular weight ardeparin, certoparin, nadroparin, logiparin, parnaparin, reviparin and tedelparin, and recombinant antithrombin III, tissue factor (TF)inhibitor-Factor Vila, such as TF mutants, anti-TF antibodies, factor VII inhibitors, thrombin inhibitors such as anti-statin factor Xa inhibitors, TAP (tick anticoagulant peptide), DX9065, lefaxin, fondaparinux, terostatin, YM-75466 and ZK-807,834, antibodies against factor IXa and Xa, protein C pathway activators and selective thrombin inhibitors such as argatroban, bivalirudin, efegatran, H376/95, hirugene, inogatran, melagatran, napsagatran, UK-156406 and ximelagatran, and chemical compounds that capture calcium ions, such as ethylenediaminetetraacetic acid (EDTA), citrate (generally sodium citrate) and oxalate.

In a particular embodiment of the method of the invention, the anticoagulant is sodium citrate.

In a particular embodiment, the anticoagulant, preferably sodium citrate, is comprised in a solution, preferably an aqueous one.

In another particular embodiment, the anticoagulant, preferably sodium citrate, is present at a concentration of between 1% and 10%, between 2% and 8%, between 2% and 6%, between 3% and 5%, between 3% and 4%, more specifically at a concentration of 3.8% (w/v), w/v referring to the weight of anticoagulant with respect to the volume of the solution in g/100 mL.

In a first step i), the method of the invention comprises obtaining a red cell free plasma from an anticoagulated blood sample of a subject.

Step i) of the method of the invention comprises treating a blood sample from a subject with an anticoagulant, wherein the meaning of anticoagulant is the same as that provided above.

In a particular embodiment, the red cell free plasma is obtained by centrifuging the anticoagulated blood sample at a speed of at least 800 g, at least 850 g, at least 900 g, at least 950 g, at least 1000 g, at least 1050 g, at least 1100 g, at least 1150 g, at least 1200 g; in particular up to: 1600 g, 1500 g, 1400 g, or 1300 g. In a particular embodiment the red cell free plasma is obtained by centrifuging the anticoagulated blood sample for at least 5 minutes, at least 6 minutes, at least 7 minutes, at least 8 minutes, at least 9 minutes, at least 10 minutes, at least 11 minutes, at least 12 minutes, at least 13 minutes, at least 14 minutes, at least 15 minutes, at least 16 minutes, at least 17 minutes, at least 18 minutes, at least 19 minutes, or at least 20 minutes; in particular up to: 45 minutes, 40 minutes, 35 minutes, 35 minutes, 30 minutes or 25 minutes. In another particular embodiment, these speeds and times are combined. A person skilled in the art will know how to combine and vary the conditions of centrifugation speed and centrifugation time by modifying them in a compensatory manner, i.e. by increasing the centrifugation force and decreasing the centrifugation time or alternatively by decreasing the centrifugation force and increasing the time to obtain red cell free plasma.

In another particular embodiment, red cell free plasma is obtained by centrifuging an anticoagulated blood sample at a speed of between 800 to 1600 g; preferably of between 1050 to 1450 g, such as at a speed of 1250 g.

In another particular embodiment, red cell free plasma is obtained by centrifuging an anticoagulated blood sample for 5-20 minutes, preferably for 5 to 11 minutes, such as for 8 minutes.

In a more particular embodiment, red cell free plasma is obtained by centrifuging an anticoagulated blood sample at a speed of between 800 to 1600 g; preferably of between 1050 to 1450 g, such as at a speed of 1250 g; and for 5-20 minutes, preferably for 5 to 11 minutes, such as for 8 minutes.

In a most particular embodiment, red cell free plasma is obtained by centrifuging an anticoagulated blood sample at a speed of 1250 g for 8 minutes.

After the centrifugation process, different layers of blood components are obtained, namely: a plasma layer at the top (supernatant) comprising the platelets and the plasma molecules; a buffy coat in the middle comprising the white blood cells; and a bottom layer comprising the red blood cells.

The red cell free plasma is obtained by isolating the supernatant and middle layers. This may be done by any means well-known to the person skilled in the art, such as by pipetting.

The term "centrifugation" refers to a method by which solids of different densities are separated from liquids by means of a rotating force, which applies a centrifugal force greater than gravity to the mixture. The solids and particles of higher density settle out. Examples of centrifugation include, but are not limited to, differential centrifugation, isopycnic centrifugation, zonal centrifugation or ultracentrifugation. In the context of the present invention differential centrifugation is preferred.

Centrifuges used to prepare RBC-free plasma can be obtained from a variety of sources, including, for example, the PLACONTM centrifuge, which is available from OCT USA Inc, Torrence.

The term "red cell free" means that the plasma contains no red blood cells or very low amounts of red blood cells. In a particular embodiment, the plasma obtained contains no red blood cells or contains less than 5% by weight, less than 4% by weight, less than 3% by weight, less than 2% by weight, less than 1% by weight of red blood cells; with respect to the total weight of the plasma.

The red cell free plasma contains a platelet concentration similar to basal blood levels. Therefore, in a particular embodiment, the red cell free plasma obtained after centrifugation contains a platelet concentration of between 100,000 and 400,000 platelets per microlitre of blood (pl/µl), more specifically between 150,000 and 350,000 platelets per microlitre of blood (pl/µl).

In a preferred embodiment, the method of the invention comprises, in step i), obtaining a plasma free of red blood cells and free of white blood cells from an anticoagulated blood sample of a subject.

The terms "leukocytes", "white blood cells" and "WBBs", used interchangeably herein, refer to blood cells that are part of the cellular effectors of the immune system. They are migratory cells that use the blood as a vehicle to gain access to different parts of the body. Leukocytes are responsible for destroying infectious agents and infected cells. Based on the microscopic characteristics of their cytoplasm (staining) and nucleus (morphology), they are divided into:
- granulocytes and polymorphonuclear cells (neutrophils, basophils and eosinophils) which have a polymorphous nucleus and numerous granules in the cytoplasm, with differential staining according to cell types; and
- agranulocytes or monomorphonuclear cells (lymphocytes and monocytes): they lack granules in the cytoplasm and have a round nucleus.

The term "red cell free and white cell free" means that the plasma does not contain red cells and white cells or contains very low amounts of red cells and white cells. In a particular embodiment, the plasma obtained contains no red blood cells nor white blood cells or contains less than 5% by weight, less than 4% by weight, less than 3% by weight, less than 2% by weight, less than 1% by weight, of each of red blood cells and white blood cells with respect to the total weight of the plasma.

The plasma free in red blood cells and white blood cells contains a platelet concentration similar to basal blood levels. Therefore, in a particular embodiment, the free plasma of red blood cells and white blood cells obtained after centrifugation contains a platelet concentration of between 100,000 and 400,000 platelets per microlitre of blood (pl/µl), more specifically between 150,000 and 350,000 platelets per microlitre of blood (pl/µl).

In said particular embodiment, the plasma free of red blood cells and free of white blood cells is obtained by centrifuging in the same manner as was done for obtaining the red cell free plasma, but then isolating only the top layer formed, i.e. not collecting the buffy coat nor the bottom layer.

In a second step ii), the method of the invention comprises concentrating the platelets and plasma molecules comprised in the plasma obtained in step i) by contacting said plasma with a hydrogel, wherein the hydrogel comprises a polymer network, and the polymer network comprises monofunctionalized N-hydroxyalkyl acrylamide monomeric units; or N-hydroxyalkyl alkylacrylamide monomeric units; or hydroxyalkyl acrylate monomeric units; or hydroxyalkyl alkylacrylate monomeric units; and is cross-linked.

Thus, the method of the present invention employs a hydrogel. The term "hydrogel" refers to a three-dimensional network of polymer chains which is capable of absorbing and retaining water to form a gel in which water is the dispersion medium. Thus, a hydrogel comprises a polymer network and water.

However, the hydrogels of the present invention are employed in a dehydrated state so that their absorbing power is maximized. In an embodiment, the hydrogels of the present invention comprise water in an amount of at most 10%, preferably at most 5%, more preferably at most 1%; by weight with respect to the total weight of the hydrogel. More preferably, the hydrogels of the present invention do not comprise water. The same weight percentages apply to any other one solvent that may be present in the hydrogel, such as any of the solvents used during its synthesis.

The hydrogel is capable of absorbing and interacting with different plasma components differently. As stated above, plasma is a cocktail of numerous molecules. It is not possible to anticipate how a given hydrogel will interact with a specific plasma, nor what the properties of the resulting hydrogel-treated plasma will be. It is also practically impossible to fully characterize these plasmas due to said molecular complexity.

Weights described herein referring to the polymer network do not comprise weights of other hydrogel components such as water.

The hydrogel employed in the method of the present invention comprises a polymer network comprising at least one of:
∘ monofunctionalized N-hydroxyalkyl acrylamide monomeric units; or
∘ monofunctionalized N-hydroxyalkyl alkylacrylamide monomeric units; or
∘ monofunctionalized hydroxyalkyl acrylate monomeric units; or
∘ monofunctionalized hydroxyalkyl alkylacrylate monomeric units.

These monomeric units will hereinafter be referred to as "the monofunctionalized acrylic monomeric units". A monofunctionalized acrylic monomeric unit stems from a monomer containing a single acrylic group. More particularly, a polymer network comprising monofunctionalized acrylic monomeric units is the result of the polymerization of a monomer mixture comprising monomers containing a single acrylic group. It is understood that, in the above four specified monomeric units and in their corresponding monomer, the acrylic group refers to the acrylamide or acrylate group.

The term "monomeric unit", or "unit" (in the context of polymers), or "repeating unit", refers to the structural motif in a polymer that stems from a monomer that has been subjected to polymerization. It therefore does not include any non-polymerizable compound which may end up in a non-recurring manner in a polymer chain, such as initiator molecules. It is distinguished from the monomer in that it is part of the polymer, whereas a monomer is an independent molecular entity which can be polymerized into a polymer. It is commonplace in the art to refer to monomeric units according to the structure of the monomer, even though the monomeric unit itself may no longer show exactly the same structure as the monomer. Thus, for instance, "acrylic monomeric unit" actually refers to a monomeric unit derived from an acrylic monomer by polymerization, even though the acrylic monomeric unit no longer comprises the C=C double bond of the acrylic group that was present in the monomer, as this is the bond which has enabled the polymerization to proceed, i.e. reacted with the incoming initiator or growing polymeric chain and then with further monomers. The skilled person is well aware of which monomers correspond to which monomeric units. Similarly, the skilled person is well aware of how to convert monomers into corresponding monomeric units by a process of polymerization.

The term "polymer", also identified by the prefix "poly", herein refers to a molecule comprising at least 10 monomeric units, such as at least 100 or at least 1000 monomeric units. Unless otherwise indicated, poly[monomeric unit X], herein refers to a molecule comprising at least 10 X monomeric units, such as at least 100 or at least 1000 X monomeric units, wherein monomeric unit X refers to a specific monomeric unit.

An N-hydroxyalkyl acrylamide moiety refers to a moiety wherein the amide nitrogen of the acrylic group is attached to an alkanol group. This moiety is termed N-hydroxyalkyl alkylacrylamide when the the C=C double bond of the acrylic group is attached to an alkyl group.

A hydroxyalkyl acrylate moiety refers to a moiety wherein the non-carbonyl ester oxygen of the acrylic group is attached to an alkanol group. This moiety is termed hydroxyalkyl alkylacrylate when the the C=C double bond of the acrylic group is attached to an alkyl group. An example of a monofunctionalized hydroxyalkyl alkylacrylate monomeric unit is a 2-hydroxyethyl methacrylate (HEMA) monomeric unit.

The term "alkyl" refers to a straight or branched, fully saturated hydrocarbon group. The alkyl is preferably a C₁₋₁₂ alkyl; more preferably a C₁₋₆ alkyl; group.

The term "hydroxyalkyl" (aka "alkanol") refers to an alkyl group as defined above substituted with at least one, preferably with one, -OH group, which more preferably is a terminal -OH group.

In the monofunctionalized N-hydroxyalkyl acrylamide, N-hydroxyalkyl alkylacrylamide, hydroxyalkyl acrylate, or hydroxyalkyl alkylacrylate monomeric units, the hydroxyalkyl group is preferably a hydroxypropyl, hydroxyethyl, or hydroxymethyl, more preferably a hydroxyethyl, group.

The alkylacrylic group in the N-hydroxyalkyl alkylacrylamide or hydroxyalkyl alkylacrylate is preferably a prop-, eth-, or meth-, more preferably a meth-, acrylic group.

In an embodiment, these hydroxyalkyl group and alkylacrylic group preferences are combined.

In a preferred embodiment, the hydrogel employed in the method of the present invention comprises a polymer network comprising at least one of:
∘ monofunctionalized N-hydroxyalkyl acrylamide monomeric units; or
∘ monofunctionalized N-hydroxyalkyl alkylacrylamide monomeric units.
More preferably, the hydrogel employed in the method of the present invention comprises a polymer network comprising monofunctionalized N-hydroxyalkyl acrylamide monomeric units, even more preferably monofunctionalized N-hydroxyethyl acrylamide (HEAA) monomeric units, yet more preferably monofunctionalized N-(2-hydroxyethyl) acrylamide monomeric units of formula (I): 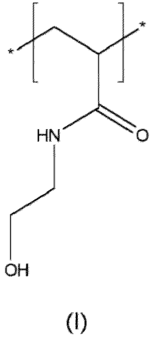 wherein * denotes a bond to the rest of the polymer network.

The polymer network comprised in the hydrogels of the present invention comprises the acrylic monomeric units, in any of the above described levels of preference, in an amount of at least 60% by weight with respect to the total weight of the polymer network. Preferably, said amount is at least 80%; more preferably said amount is at least 90%; by weight with respect to the total weight of the polymer network.

The maximum possible amount by weight of the monofunctionalized acrylic monomeric units with respect to the total weight of polymer network is dictated by the amount of other components comprised in the polymer network.

In an embodiment, the maximum possible amount by weight of the monofunctionalized acrylic monomeric units with respect to the total weight of polymer network is dictated by the amount of bifunctionalized acrylic monomeric units, and also the amount of initiator if it is present. Thus, at least 90% by weight of monofunctionalized acrylic monomeric units becomes 90% to 95.0% by weight of the monofunctionalized acrylic monomeric units if the embodiment requires 2.6% by weight of bifunctionalized acrylic monomeric units and 2.4% of initiator.

In an embodiment, the polymer network comprised in the hydrogels of the present invention comprises the acrylic monomeric units in an amount of from 80% to 99.98% by weight with respect to the total weight of the polymer network. Preferably, said amount is from 85% to 99%; more preferably said amount is from 90% to 95%; by weight with respect to the total weight of the polymer network.

The polymer network comprised in the hydrogels of the invention is cross-linked. Cross-linking may be achieved by different manners known to the skilled person, e.g. by chemical means, such as by the inclusion of bifunctionalized acrylic monomers in the polymerisation reaction; or by physical means, such as by freeze-thawing.

In a preferred embodiment, cross-linking is achieved by the inclusion of bifunctionalized acrylic monomers in the polymerisation reaction. Therefore, in a preferred embodiment, the polymer network comprises bifunctionalized acrylic monomeric units. A bifunctionalized acrylic monomeric unit stems from a monomer containing two or more, preferably two, acrylic groups. More particularly, a polymer network comprising bifunctionalized acrylic monomeric units is the result of the polymerization of a monomer mixture comprising monomers containing two or more, preferably two, acrylic groups.

Non-limiting examples of bifunctionalized acrylic monomeric units are ethylene glycol dimethacrylate, 1,3-butanediol diacrylate, 1,4-butanediol diacrylate, 1,6-hexanediol diacrylate, ethylene glycol diacrylate, fluorescein O,O'-diacrylate, glycerol 1,3-diglycerolate diacrylate, pentaerythritol diacrylate monostearate, 1,6-hexanediol ethoxylate diacrylate, 3-hydroxy-2,2-dimethylpropyl 3-hydroxy-2,2-dimethylpropionate diacrylate, bisphenol A ethoxylate diacrylate, di(ethylene glycol) diacrylate, neopentyl glycol diacrylate, propylene glycol glycerolate diacrylate, tetra(ethylene glycol) diacrylate, 1,3-butanediol dimethacrylate, 1,4-butanediol dimethacrylate, 1,6-hexanediol dimethacrylate, bisphenol A dimethacrylate, diurethane dimethacrylate, fluorescein O,O'-dimethacrylate, glycerol dimethacrylate, bisphenol A ethoxylate dimethacrylate, bisphenol A glycerolate dimethacrylate, di(ethylene glycol) dimethacrylate, tetraethylene glycol dimethacrylate, tri(ethylene glycol) dimethacrylate or triethylene glycol dimethacrylate, or combinations thereof.

Preferably, the polymer network comprises bifunctionalized acrylic monomeric units wherein the acrylic group is an acrylate or acrylamide group; even more preferably wherein the acrylic group is an acrylamide group, such as in N,N-Methylenebis(acrylamide) (MBAA).

More preferably, the acrylic groups in the bifunctionalized acrylic monomeric unit are connected through an alkyl diradical linker, wherein the term "alkyl" is as defined above.

Even more preferably, said alkyl linker is a propyl, ethyl, or methyl, more preferably a methyl linker, such as in MBAA.

More preferably, it is the non-carbonyl ester oxygen of any acrylate group or the amide nitrogen of any acrylamide group which is connected to the alkyl linker, such as in MBAA.

Most preferably, the polymer network comprises MBAA monomeric units.

In a preferred embodiment, the polymer network comprises an amount of between 0.01% and 10% by weight of bifunctionalized acrylic monomeric units with respect to the total weight of the polymer network; preferably said amount is of between 0.10% and 5.0%, more preferably said amount is of between 2.0% and 3.0%. This applies to any level of preference of the bifunctionalized acrylic monomeric units as described above.

In an embodiment, the polymer network comprises, and more particularly consists of:
- an amount of at least 60%, preferably at least 80%, more preferably at least 90%; by weight of the monofunctionalized acrylic monomeric units, more particularly in any preferred form of these as described above; and
- an amount of between 0.01% and 10%, preferably of between 0.10% and 5.0%, more preferably of between 2.0% and 3.0%; by weight of bifunctionalized acrylic monomeric units, preferably in any preferred form of these as described above;
with respect to the total weight of the polymer network.

In a particular embodiment, the monofunctionalized acrylic monomeric units are N-(2-hydroxyethyl) acrylamide monomeric units.

In another particular embodiment, the bifunctionalized acrylic monomeric units are MBAA monomeric units.

In a more particular embodiment, the monofunctionalized acrylic monomeric units are N-(2-hydroxyethyl) acrylamide monomeric units and the bifunctionalized acrylic monomeric units are MBAA monomeric units.

The hydrogels of the present invention are prepared by polymerization of a mixture of monomers. The polymerization reaction may be initiated with or without the addition of an initiator compound (aka initiator) to the monomer mixture.

Examples of polymerization initiated without the addition of an initiator compound are those initiated by subjecting the monomer mixture to high-energy ionizing radiation (such as gamma rays or electrons).

More commonly, however, the polymerization reaction is initiated by addition of an initiator compound (aka initiator) to the monomer mixture followed by activation of said initiator.

Thus, in an embodiment, the polymer network further comprises initiator.

Polymerization reactions initiated by an initiator include thermal- or photo-polymerization.

Thermal polymerization is carried out by subjecting the monomer and initiator mixture to a temperature at which homoleptic cleavage of a bond in the initiator molecule occurs, thus leading to radical initiator fragments which react with the monomers to initiate polymerization. Some initiators require elevated temperatures for cleavage (such as AIBN), for example of from 30 to 100°C, while others can generate said radicals at room temperature (certain peroxides). The specific temperature required to initiate thermal polymerization thus depends on the specific initiator employed. Said temperatures are well-known to the person skilled in the art.

Examples of thermal polymerization initiator types are peroxides, hydroperoxides, azobis(alkyl- or cycloalkyl-nitriles), persulfates, percarbonates or mixtures thereof. Specific examples are benzoylperoxide, tert-butyl peroxide, di-tert-butyl-diperoxyphthalate, tert-butyl hydroperoxide, azo-bis(isobutyronitrile) (AIBN), 1,1-azodiisobutyramidine, 1,1'-azobis (1-cyclohexanecarbonitrile), 2,2'-azo-bis(2,4-dimethylvaleronitrile), (2,2'-azobis (2-amidinopropane) dihydrochloride. Preferably, the thermal polymerization initiator is (2,2'-azobis (2-amidinopropane) dihydrochloride; AIBN; or a persulfate, such as ammonium persulfate (APS) or potassium persulfate (KPS), optionally combined with a catalyst such as N,N,N',N'-tetramethylethylenediamine (TEMED).

Photopolymerization is carried out by subjecting the monomer and initiator mixture to light radiation, thus exciting the initiator to higher electronic levels and converting it into free radicals, cations or anions that can react with the monomers to initiate polymerization. Different initiators respond to different light radiation. Commonly, the light radiation is UV or visible light radiation. For instance, Eosin Y responds to visible radiation, while 2-Hydroxy-4'-(2-hydroxyethoxy)-2-methylpropiophenone (aka Irgacure 2959) responds to UV radiation.

Examples of photoinitiators are benzoin methyl ether, 2,4,6-trimethylbenzoyldiphenylophosphine oxide, bis-(2,6-dichlorobenzoyl)-4-N-propylphenylphosphine oxide, bis-(2,6-dichlorobenzoyl)-4-N-butylphenylphosphine oxide, diethoxyacetophenone, 2-Hydroxy-4'-(2-hydroxyethoxy)-2-methylpropiophenone, 1-hydroxycyclohexyl phenyl ketone, Germane-based Norrish Type I compounds or persulfates. Preferably, the photoinitiator is 2-Hydroxy-4'-(2-hydroxyethoxy)-2-methylpropiophenone.

Polymerization reaction times vary within wide limits, but can conveniently be, for example, from 1 to 48 hours.

Polymerization reactions are commonly, although not necessarily, carried out in a solvent. Examples of suitable solvents include, without limitation, water, tetrahydrofuran, glycols such as alkylene glycols, ketones such as acetone, ethyl acetate, methylene chloride, alcohols such as ethanol or isopropanol, 1-methyl-2-pyrrolidone, N,N-dimethylpropionamide, dimethyl formamide, DMSO or mixtures thereof. Preferably, the solvent is a mixture, preferably an aqueous mixture, of an alkylene glycol and an alcohol, more particularly of ethylene glycol and ethanol. Also preferably, the solvent is aqueous, more preferably it is water.

In an embodiment, the polymer network comprises an amount of initiator of between 0.01% and 10% by weight with respect to the total weight of the polymer network; preferably said amount is of between 0.10% and 5.0%, more preferably said amount is of between 2.0% and 3.0%.

Similarly to the above discussed structural difference between a monomer and its corresponding monomeric unit, which is a consequence of reaction, the structure of the initiator in the polymer network is not identical to the structure of the initiator in the monomer and initiator mixture which is to be subjected to polymerization. It is common general knowledge to the person skilled in the art how the initiator structure is altered as a consequence of the polymerization reaction.

In an embodiment, the polymer network comprises, and more particularly consists of:
- an amount of at least 60%, preferably at least 80%, more preferably at least 90%; by weight of the monofunctionalized acrylic monomeric units, more particularly in any preferred form of these as described above;
- an amount of between 0.01% and 10%, preferably of between 0.10% and 5.0%, more preferably of between 2.0% and 3.0%; by weight of bifunctionalized acrylic monomeric units, preferably in any preferred form of these as described above; and
- an amount of between 0.01% and 10%, preferably of between 0.10% and 5.0%, more preferably of between 2.0% and 3.0%; by weight of initiator, preferably in any preferred form of it as described above;
with respect to the total weight of the polymer network.

In a particular embodiment, the monofunctionalized acrylic monomeric units are N-(2-hydroxyethyl) acrylamide monomeric units.

In another particular embodiment, the bifunctionalized acrylic monomeric units are MBAA monomeric units.

In another particular embodiment, the initiator is 2-Hydroxy-4'-(2-hydroxyethoxy)-2-methylpropiophenone. In an alternative particular embodiment, the initiator is a mixture of APS and TEMED.

In a more particular embodiment, the monofunctionalized acrylic monomeric units are N-(2-hydroxyethyl) acrylamide monomeric units; the bifunctionalized acrylic monomeric units are MBAA monomeric units; and the initiator is 2-Hydroxy-4'-(2-hydroxyethoxy)-2-methylpropiophenone.

In an alternative more particular embodiment, the monofunctionalized acrylic monomeric units are N-(2-hydroxyethyl) acrylamide monomeric units; the bifunctionalized acrylic monomeric units are MBAA monomeric units; and the initiator is a mixture of APS and TEMED.

It is understood that in any of the herein described embodiments relating to the polymer network, the different components are chosen such as to not exceed 100% by weight with respect to the total weight of the polymer network.

In an embodiment, the hydrogel comprising this polymer network is obtained by mixing the corresponding monomers and the reducing agents used as initiators to promote a redox reaction-based gel formation. Preferably, said mixture is carried out in an aqueous solvent, more preferably in water.

The person skilled in the art knows how to translate the composition of the hydrogels into the corresponding non-polymerized monomer mixture. Thus, in an embodiment, every embodiment provided herein describing the nature and amounts of the monomeric units and initiator comprised in the polymer network comprised in the hydrogels used in the present invention refers rather to the nature and amounts of the corresponding monomers and initiator comprised in the non-polymerized mixture that is to be subjected to polymerization with respect to the combined weight of said monomers and initiator, i.e. excluding solvent. Thus, by way of example, when the polymer network comprised in the hydrogel used in the present invention comprises an amount of from 90% to 95% of N-(2-hydroxyethyl) acrylamide monomeric units by weight with respect to the total weight of the polymer network, this automatically generates an embodiment wherein the mixture that is to be subjected to polymerization comprises an amount of from 90% to 95% of N-(2-hydroxyethyl) acrylamide monomers with respect to the combined weight of monomers and initiator in said mixture.

The hydrogel used in the method of the present invention is preferably used in the form of granules. Hydrogel granules can be prepared by granulating the hydrogel material obtained from the polymerization reaction to obtain a granule mixture. This can be achieved by means well-known to the person skilled in the art, such as by mechanical methods, e.g. by grinding such as by mortar and pestle, or milling such as by wet milling; preferably granulation is performed by mortar and pestle grinding.

In a preferred embodiment, the hydrogel is dried prior to contacting it with the plasma obtained in step i) of the method of the present invention. By carrying out this step, the absorption power of the hydrogel is maximized. Drying can be achieved by heating, typically to a temperature which achieves evaporation of the solvent that was employed in the polymerization reaction which led to the formation of the hydrogel. Drying can be performed prior to or after granulation. The drying is preferably performed until a dehydrated hydrogel as was described hereinabove is obtained.

In an embodiment, the hydrogel granules possess a size of 0.5-1 mm, which refers to the longest diameter of the granule. Specific granule sizes can be obtained by screening out, such as by sieving, the desired particle size from the granule mixture obtained after granulating the hydrogel as described hereinabove. In a preferred embodiment, the granules are dried prior to screening.

In an embodiment, the red blood cell-free or red blood cell-free and white blood cell-free plasma obtained in step (i) is placed in contact with the hydrogel for 2-60 minutes, preferably for 5-40 minutes.

In an embodiment, the red blood cell-free or red blood cell-free and white blood cell-free plasma obtained in step (i) is placed in contact with the hydrogel at a ratio of at least 0.1 g of hydrogel per 4 mL of plasma, preferably at least 0.40 g of hydrogel per 4 mL of plasma, more preferably at least 0.45 g of hydrogel per 4 mL of plasma, even more preferably at least 0.50 g of hydrogel per 4 mL of plasma; such as from any of these values up to 1 g of hydrogel per 4 mL of plasma, preferably up to 0.8 g of hydrogel per 4 mL of plasma; more preferably up to 0.6 g of hydrogel per 4 mL of plasma. More preferably, these ratios refer to hydrogel granules with a diameter between 0.5-1 mm and with a water content of at most 1% by weight with respect to the total weight of said hydrogel.

In a preferred embodiment, the above times and ratios are combined.

In another particular embodiment, the method of the invention comprises an additional third step iii) comprising separating the concentrated plasma from the hydrogel, such as by filtering the hydrogel-plasma mixture using a filter with a pore size that is capable of retaining the hydrogel.

To this effect, and as an expert in the field may know, the pore size of the filter will vary depending on the particle size of the hydrogels used. The filter will have a pore size that is capable of retaining the hydrogel, e.g. a pore size of 100µm is suitable for retaining hydrogel granules with a longest diameter between 0.5-1 mm.

In a particular embodiment, the filter used in the method of the invention has a pore size of at least 5 µm.

In another particular embodiment, the filtration step is facilitated by a centrifugation process. In said particular embodiment, the centrifugation is carried out at a speed of at least 200 g, at least 300 g, at least 400 g, at least 500 g, at least 600 g, at least 700 g, at least 750 g, at least 800 g, at least 850 g, at least 850 g, at least 900 g, at least 950 g, in particular up to 1000 g. In another embodiment, the centrifugation is carried out for at least 1 minutes, at least 2 minutes, at least 3 minutes, at least 4 minutes, at least 5 minutes, at least 6 minutes, at least 7 minutes, at least 8 minutes, at least 9 minutes, at least 10 minutes, at least 11 minutes, at least 12 minutes, at least 13 minutes, at least 14 minutes, at least 15 minutes. In another embodiment, these speeds and times are combined. A person skilled in the art will know how to combine and vary the conditions of centrifugation speed and centrifugation time by modifying them in a compensatory manner.

In another particular embodiment, the centrifugation process to facilitate the filtration step is carried out at a speed of between 200 and 1000 g; preferably of between 300 and 700 g, such as at a speed of 500 g.

In another particular embodiment, the centrifugation process to facilitate the filtration step is carried out for 1-15 minutes, preferably for 1-5 minutes, such as for 2 minutes.

In a more particular embodiment, the centrifugation process to facilitate the filtration step is carried out at a speed of between 200 and 1000 g; preferably of between 300 and 700 g, such as a speed of 500 g; and for 1-15 minutes, preferably for 1-5 minutes, such as for 2 minutes.

In a most particular embodiment, the centrifugation process to facilitate the filtration step is carried out at a speed of 500 g for 2 minutes.

In another particular embodiment, the method of the invention further comprises an additional step of activating the platelets obtained in the concentrated, and optionally separated, plasma.

The term "platelet activation" refers herein to the process by which platelets present in plasma aggregate to cause clot formation and promote haemostasis and healing by, among other processes, secretion of growth factors or intercellular mediators from cytoplasmic granules. Platelets can be activated by a large number of substances, to which they react within seconds. When exposed to these agonists, platelets exhibit different responses, which are closely associated in vivo, but which, in vitro, can be studied separately: adhesion and shape change, aggregation, secretion and expression of procoagulant activity.

Activated platelets change their shape by becoming more spherical, forming pseudopods on their surface. They thus take on a stellate shape. After activation, granule secretion occurs. Platelets contain alpha granules and dense granules. Activated platelets excrete the contents of these granules into their canalicular systems and into the surrounding blood or plasma. There are two types of granules: (i) dense granules (containing ADP or ATP, calcium, and serotonin) and (ii) granules-α (containing platelet factor 4, transforming growth factor beta 1 (TGF beta 1), platelet-derived growth factor, fibronectin, B-thromboglobulin, vWF, fibrinogen, and clotting factors (factor V and VIII), among others.

In one particular embodiment the platelets are activated by a substance selected from calcium chloride, thrombin and collagen. In another particular embodiment the platelets are mechanically activated by contact with glass microspheres.

As mentioned, in a particular embodiment, platelets are activated by a substance selected from calcium chloride, thrombin and collagen, preferably calcium chloride. Activation of the platelets is carried out by addition of that substance to the plasma obtained after the concentration, and optionally separation, step. In a more preferred embodiment, platelet activation is carried out by the addition of a preferably aqueous calcium chloride solution of at least 5%, such as 5 to 30%, more preferably 10% by weight of calcium chloride relative to the volume of the solution (w/v in g/100 mL).

In a particular embodiment, platelet activation is achieved by the addition of CaCl₂ solution as described above, at a concentration between 10 µl /ml of plasma and 60 µl /ml of plasma, between 15 µl /ml of plasma and 50 µl /ml of plasma, more particularly approximately 20 µl /ml of plasma.

In a particular embodiment, after the red cell-free or red and white cell-free plasma has been obtained, but before concentration, the platelet and total protein concentrations in the red cell-free or red and white cell-free plasma are measured to ensure that they remain at similar values to those of the anticoagulated blood from which the plasma is obtained. Similar value means values equal to those of the anticoagulated blood from which such plasma is obtained or which differ by up to 1%, 2%, 3%, 4%, 5%, 6%, 7%, 8%, 9% or up to 10% from the values of the anticoagulated blood from which such plasma is obtained.

To obtain the platelet and protein concentration factors of PRP relative to blood levels, the respective analyses of blood samples and their corresponding PRP samples were performed after processing according to the invention. Measurement of platelet concentration can be performed by a haematology analyser based on fluorescence flow cytometry technology (Sysmex XS-1000i; Kobe, Japan). Samples were measured using the Complete Blood Count with Differential test. Total protein concentration can be measured by a Cobas c 501 analyser (Roche, Basel, Switzerland).

In a further particular embodiment, once the concentrated and separated plasma has been obtained, the platelet and total protein concentrations are again measured. The platelet and total protein concentrations can be measured as described above. The purpose of this second measurement is to check that the platelet and total protein concentrations have increased with respect to the concentrations measured in the preconcentration RBC-free or RBC/WBC-free plasma or with respect to the anticoagulated blood from which the plasma is obtained.

### Plasma enriched in platelets and plasma molecules

In a second aspect, the invention relates to a plasma enriched in platelets and plasma molecules obtained by the method of the invention.

In a particular embodiment, the platelet and total protein concentration of the enriched plasma is higher, preferably at least 1.5 times higher than that of the anticoagulated blood sample of step i).

The plasma obtained by the method of the invention contains a platelet concentration higher than that of the anticoagulated blood sample of step i). In a particular embodiment, the plasma obtained by the method of the invention contains a platelet concentration between 1.2 and 5 times higher than that of the anticoagulated blood sample of step i).

In another particular embodiment, the plasma obtained by the method of the invention contains a platelet concentration between 1.5 and 3, between 1.7 and 2 times higher than the levels in the anticoagulated blood sample of step i).

In a particular embodiment the concentration of platelets in the enriched plasma is between 200,000 and 600,000 platelets per microlitre of plasma (pl/µl), between 225,000 and 550,000 platelets per microlitre of plasma (pl/µl), between 250,000 to 500,000 platelets per microlitre of plasma (pl/µl), from 300,000 to 450,000 platelets per microlitre of plasma (pl/µl), more preferably from 225,000 to 525,000 platelets per microlitre of plasma (pl/µl).

In another particular embodiment, the plasma obtained by the method of the invention contains a higher total protein concentration than that of the anticoagulated blood sample of step i). In a particular embodiment, the total protein concentration of the plasma obtained by the method of the invention is between 1.2 and 5 times higher than that of the anticoagulated blood sample of step i), between 1.5 and 3 times that of the anticoagulated blood sample of step i), between 1.7 and 2.3 times that of the anticoagulated blood sample of step i).

In a particular embodiment, the total protein concentration of the plasma obtained by the method of the invention is between 10 and 15 g /decilitre of plasma, between 11 and 13 g/ decilitre of plasma, more preferably from 11.5 to 12.5 g/decilitre of plasma.

Total protein measurements are performed prior to platelet activation and degranulation. Hence, the above values refer to the content of plasma proteins only, and not to plasma plus platelet proteins.

In a preferred embodiment, the plasma obtained by the method of the invention contains:
- a platelet concentration between 1.2 and 5, particularly between 1.5 and 3, more particularly between 1.7 and 2, times that of the anticoagulated blood sample of step i); and
- a total protein concentration between 1.2 and 5, particularly between 1.5 and 3, more particularly between 1.7 and 2.3, times that of the anticoagulated blood sample of step i).

In an embodiment, the ratio of platelet concentration to total protein concentration (total protein concentration being representative of plasmatic protein concentration) in the plasma obtained by the method of the invention maintains the equilibrium found in the anticoagulated blood sample of step i), i.e. the ratio of platelet concentration to total protein concentration in the plasma obtained by the method of the invention differs from the same ratio in the anticoagulated blood sample of step i) by no more than 20%, preferably no more than 10%, more preferably no more than 5%.The plasma obtained by the method of the invention, contains an albumin concentration higher than that of the anticoagulated blood sample of step i) of the method of the invention. More specifically, the plasma obtained by the method of the invention, contains an albumin concentration between 1.2 and 5 times higher than that of anticoagulated blood sample of step i) of the method of the invention, between 1.5 and 3 times higher than that of anticoagulated blood sample of step i), between 1.7 and 2.2 times higher than that of anticoagulated blood sample of step i).

Measurement of platelet concentration and total protein concentration can be measured as was described above. Albumin concentration can be measured by a Cobas c 501 analyser (Roche, Basel, Switzerland).

The plasma obtained by the method of the invention has an HGF concentration higher than that of the anticoagulated blood sample of the step i) of the method of the invention. In a particular embodiment, the plasma obtained by the method of the invention has a HGF concentration at least 1.5 times higher, preferably at least 2 times higher, more preferably at least 2.05 times higher than that of the anticoagulated blood sample of the step i) of the method of the invention, such as from any of these values up to 2.36 times higher.

The plasma obtained by the method of the invention has an IGF-I concentration higher than that of the anticoagulated blood sample of step i) of the method of the invention, In another particular embodiment, the plasma obtained by the method of the invention has an IGF-I concentration at least 1.5 times higher, preferably at least 2 times higher, more preferably at least 2.25 times higher, even more preferably at least 2.5 times higher, yet more preferably at least 2.6 times higher than that of the anticoagulated blood sample of step i) of the method of the invention, such as from any of these values up to 3.03 times higher.

The IGF-1 range in blood varies according to the age of the subject. Considering all ages, the range of IGF-1 in blood varies between 30 and 640 ng/mL.

The concentration of both platelet and plasmatic molecules such as IGF-1, HGF, PDGF-AB, TGFβ1 and VEGF, can be analysed as follows. First, plasma samples are activated by adding a 10% aqueous CaCl₂ solution at a ratio of 20µL per 1 mL of plasma. Then, the concentration of those molecules is measured by commercially available enzyme-linked immunosorbent assay kit (ELISA) following manufacturers' instructions (R&D Systems, Minneapolis, USA).

In the context of the present invention, the expression "superior" refers to:(i)a concentration of the compounds being tested higher than that of the blood of a control subject, which corresponds to the anticoagulated blood of step i) of the method of the invention, or (ii) a concentration of the compounds being tested higher than that of a reference PRP, in the present invention, sPRP or WOPRP. The different methods of preparation of PRPs have an impact on the preservation capacity and concentration of the different components of the anticoagulated blood. In this regard, it has been observed that the plasma obtained according to the present invention possesses surprisingly high levels of certain growth factors, such as IGF-I.

The term IGF-I refers to insulin-like growth factor type 1, also known as somatomedin C or mechano growth factor (MGF). In a particular embodiment, IGF-I is the protein encoded by the human gene shown in the Ensemble database under accession number ENSG00000017427 (Ensembl version 106, April 2022). The protein encoded by this gene is similar in function and structure to insulin and is a member of a family of proteins involved in mediating growth and development. The encoded protein is processed from a precursor, bound to a specific receptor and secreted.

### Medical uses

In a third aspect, the invention relates to a plasma enriched in platelets and plasma molecules according to the second aspect of the invention (hereinafter "the plasma of the invention") for use in medicine.

As mentioned above, the plasma obtained by the method of the present invention possesses both concentrated levels of platelets (and therefore platelet growth factors) and non-platelet biomolecules, especially non-platelet growth factors, including a high content of IGF-I or/and HGF.

The higher concentration of platelets improves the healing properties of the plasma compared to plasmas in which the platelets are not concentrated, such as plasma isolated directly from blood. Platelets function as exocytotic cells, secreting a variety of effector molecules at sites of injury.

In relation to the plasma of the invention, the inventors have surprisingly discovered that by the specific method of plasma processing according to the present invention, the concentration of specific non-platelet biomolecules, especially growth factors such as IGF-I or/and HGF is greatly increased and more importantly the regenerative potential of the plasma is greatly enhanced.

Thus, in a preferred embodiment, the present invention relates to a plasma enriched in platelets and plasma molecules according to the second aspect of the invention for use in regenerative medicine.

The use in regenerative medicine refers more particularly to the treatment of injured tissue in a subject.

The present invention also relates to a method of treating injured tissue in a subject in need thereof, comprising administering the plasma of the invention to the subject.

The present invention also relates to the use of the plasma of the present invention in the preparation of a medicament for the treatment of injured tissue.

As used herein, the term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. Accordingly, the "treatment" of a disorder or disease may also refer to an amelioration of the disorder or disease, which may, e.g., lead to a halt in the progression of the disorder or disease or a delay in the progression of the disorder or disease. Such a partial or complete response may be followed by a relapse. It is to be understood that a subject/patient may experience a broad range of responses to a treatment.

As used herein, the term "injured" is used in its ordinary sense to refer to any tissue damage, but preferably a wound, trauma or ulcer.

In a preferred embodiment, the tissue is musculoskeletal tissue.

In a particular embodiment, it is bone or cartilage.

In another particular embodiment, the injured tissue is a soft tissue. Soft tissue refers to all the tissue in the body that is not hardened by the processes of ossification or calcification such as bones and teeth. In a more particular embodiment, the injured tissue is selected from the group consisting of connective tissue, cardiac muscle, skeletal muscle, brain tissue, corneal tissue, nerve tissue and vascular tissue.

Examples of specific disease states that can be treated with the plasma of the present invention are articular cartilage injury, skeletal muscle injury, ligament injury, osteoporosis, age-related wasting, or muscular dystrophy.

In another particular embodiment, the plasma of the present invention is used in dentistry, in particular after oral surgery.

The plasma of the invention can be administered at any suitable dose. In some embodiments, the dose may be between 1 ml and 20 ml. The dose is generally determined according to the specific medical procedure followed, the condition treated and the patient's profile.

The plasma of the invention can be administered orally and parenterally, such as intravenously (iv), intraperitoneally (ip), subcutaneously (sc), intramuscularly (im), rectally, topically, ophthalmically, nasally and transdermally. The plasma of the invention can be administered to a subject in need thereof by injection using a syringe or a catheter. The plasma of the invention can also be administered via a dermal patch, a spray device or in combination with an ointment or bone graft. It can also be used as a coating on sutures, stents, screws, plates or other implantable medical devices. Plasma of the invention formulated as gels or other viscous fluids can be difficult to administer through a needle or syringe. Therefore, in variations where the use of a needle or syringe is desired, it may be desirable to add a gelling and/or hardening agent to the plasma of the invention in situ.

The plasma enriched in platelets and plasma molecules can function as a scaffold and can be used as an implant per se, to provide mechanical support to an injured site in situ and/or to provide a matrix within which cells from the injured site can proliferate and differentiate. The site of administration of the PRP composition is typically at or near the site of tissue damage. The site of tissue damage is determined by well-established methods including imaging studies and patient feedback or a combination thereof. In some examples, the plasma of the invention can be administered to the damaged connective tissue in or around the affected joints.

On the other hand, IGF-I and HGF, which are especially concentrated in the plasma of the invention, have shown great utility in inflammatory, allergic or fibrotic diseases.

In another aspect, the invention relates to the plasma enriched in platelets and plasma molecules according to the second aspect of the invention for use in the treatment of inflammatory diseases.

The present invention also relates to a method of treating inflammatory diseases in a subject in need thereof, comprising administering a plasma of the invention to the subject.

The present invention also relates to the use of the plasma of the present invention in the preparation of a medicament for the treatment of inflammatory diseases.

Examples of specific inflammatory diseases that can be treated with the plasma of the present invention are rheumatic diseases, such as osteoarthritis or rheumatoid arthritis.

In a particular embodiment, the plasma of the present invention is administered to the same patient from which the anticoagulated blood sample of step i) was obtained. In other words, in a particular embodiment the plasma of the present invention that is administered to the subject is autologous.

In another particular embodiment, the plasma of the present invention is administered to a different patient from which the anticoagulated blood sample of step i) was obtained. In other words, in a particular embodiment, the plasma of the present invention that is administered to the subject comes from a donor, i.e. is allogeneic.

In another aspect, the invention relates to the cosmetic use of the plasma obtained by the method of the present invention. Particular cosmetic uses are the treatment of skin wrinkles, stretch marks or dark circles under the eyes.

The term "cosmetic" as used herein refers to articles (excluding soap) intended to be rubbed, poured, sprinkled, or sprayed on, introduced into, or otherwise applied to the human body or any part thereof for cleansing, beautifying, promoting attractiveness, or altering the appearance, and articles intended for use as a component of any such articles.

The plasma of the present invention may be added to a wide variety of products for cosmetic application, including makeup, creams for cleansing, protecting, treating, or caring for the skin, in particular, the face, hands, and feet (e.g., day and night creams, makeup removal creams, foundation creams and sunscreens), liquid foundations, makeup removal lotions, protective or skin-care body lotions, sunscreen lotions, skin care lotions, gels, or foams, such as cleansing, sunscreen, and artificial tanning lotions, bath preparations, deodorant compositions, after-shave gels or lotions, depilatory creams, and compositions used for insect stings and against pain. The plasma of the invention may take any of a wide variety of forms, and include, for example dressings, lotions, solutions, sprays, creams, gels, ointments, or the like.

Lastly, the invention also relates to the *in vitro* use of a hydrogel for concentrating blood or plasma, wherein the hydrogel is as defined in any embodiment of the first aspect of the invention.

Preferably, the plasma is obtained from blood by removal of RBCs and optionally also removal of WBCs, such as by a method according to step i) as described in any embodiment of the first aspect of the invention.

Preferably, the blood sample is anticoagulated as described in any embodiment of the first aspect of the invention.

The use comprises contacting the blood or plasma with the hydrogel such as described at step ii) as described in any embodiment of the first aspect of the invention, and then optionally separating the hydrogel from the concentrated blood or plasma, such as by filtering the hydrogel-blood or hydrogel-plasma mixture using a filter with a pore size that is capable of retaining the hydrogel such as described at step iii) as described in any embodiment of the first aspect of the invention.

In further aspects, the present invention relates to:
1. An *in vitro* method for producing a plasma enriched in platelets and plasma molecules comprising:
   (i) obtaining a red blood cell-free plasma from an anticoagulated blood sample of a subject, wherein the plasma comprises platelets and plasma molecules; and
   (ii) concentrating the platelets and plasma molecules comprised in the plasma obtained in step i) by contacting said plasma with a hydrogel, wherein the hydrogel comprises a polymer network, and the polymer network comprises monofunctionalized N-hydroxyalkyl acrylamide monomeric units; or N-hydroxyalkyl alkylacrylamide monomeric units; or hydroxyalkyl acrylate monomeric units; or hydroxyalkyl alkylacrylate monomeric units; and is cross-linked.
2. Method according to aspect 1, wherein in step i) the plasma obtained from the anticoagulated blood sample of a subject is free of red blood cells and white blood cells.
3. Method according to aspect 1, wherein the red blood cell free plasma of step i) is obtained by centrifugation at a speed of between 800 to 1600 g for a time of 5 to 20 minutes from the anticoagulated blood sample, and isolating the supernatant and buffy coat layers from the centrifuged blood sample.
4. Method according to aspect 2, wherein the red blood cell free and white blood cell free plasma of step i) is obtained by centrifugation at a speed of between 800 and 1600 g for a time of 5 to 20 minutes from the anticoagulated blood sample, and isolating the supernatant layer from the centrifuged blood sample.
5. Method according to any one of aspects 1 to 4, wherein the polymer network comprised in the hydrogel used in step (ii) comprises monofunctionalized N-hydroxyalkyl acrylamide monomeric units or monofunctionalized N-hydroxyalkyl alkylacrylamide monomeric units.
6. Method according to aspect 5, wherein the polymer network comprised in the hydrogel used in step (ii) comprises monofunctionalized N-hydroxyalkyl acrylamide monomeric units.
7. Method according to aspect 6, wherein the monofunctionalized N-hydroxyalkyl acrylamide monomeric units are N-hydroxyethyl acrylamide (HEAA) monomeric units.
8. Method according to any one of aspects 1 to 7, wherein the polymer network comprised in the hydrogel used in step (ii) comprises the monofunctionalized monomeric units in an amount of at least 60% by weight with respect to the weight of the polymer network.
9. Method according to any one of aspects 1 to 8, wherein the polymer network comprised in the hydrogel used in step (ii) comprises bifunctionalized acrylic monomeric units.
10. Method according to aspect 9, wherein the bifunctionalized acrylic monomeric units are N,N-Methylenebis(acrylamide) (MBAA) monomeric units.
11. Method according to any one of aspects 9 or 10, wherein the polymer network comprised in the hydrogel used in step (ii) comprises the bifunctionalized acrylic monomeric units in an amount of between 0.01% and 10% by weight with respect to the weight of the polymer network.
12. Method according to any one of aspects 1 to 11, wherein the polymer network comprises initiator, preferably the initiator is a mixture of APS and TEMED.
13. Method according to any one of aspects 1 to 12, wherein the red blood cell-free plasma obtained in step (i) is placed in contact with the hydrogel for 2-60 minutes.
14. Method according to any one of aspects 1 to 13, wherein the method further comprises an additional step comprising filtering the hydrogel-plasma mixture using a filter with a pore size that is capable of retaining the hydrogel.
15. Method according to aspect 14, wherein the filter used in step iii) has a pore size of at least 5 µm.
16. Method according to any one of aspects 14 or 15, wherein the filtration is facilitated by centrifugation at a speed of between 200 and 1000 g for 1-15 minutes.
17. Method according to any one of aspects 1 to 16, further comprising the step of activating the platelets obtained in the filtered plasma.
18. Method according to aspect 17, wherein the activation of platelets is performed by the addition of a compound selected from calcium chloride, thrombin and collagen.
19. Method according to aspect 18, wherein the activation of platelets is performed by the addition of calcium chloride.
20. Plasma enriched in platelets and plasma molecules obtained by the method according to any one of aspects 1 to 19.
21. Plasma enriched in platelets and plasma molecules according to aspect 20, wherein the platelet and total protein concentration of the enriched plasma is at least 1.5 times higher than that of the anticoagulated blood sample of step i).
22. Plasma enriched in platelets and plasma molecules according to aspect 20, wherein the concentration of albumin is at least 1.5 times higher than that of the anticoagulated blood sample of step i).
23. Plasma enriched in platelets and plasma molecules according to aspect 20, wherein the concentration of HGF is higher than that of the anticoagulated blood sample of step i), preferably at least 1.5 times higher than that of the anticoagulated blood sample of step i), more preferably at least 2 times higher than that of the anticoagulated blood sample of step i).
24. Plasma enriched in platelets and plasma molecules according to aspect 20, wherein the concentration of IGF-I is higher than that of the anticoagulated blood sample of step i), preferably at least 1.5 times higher than that of the anticoagulated blood sample of step i), more preferably at least 2 times higher than that of the anticoagulated blood sample of step i).
25. Plasma enriched in platelets and plasma molecules according to any one of aspects 20 to 24 for use in medicine.
26. Plasma enriched in platelets and plasma molecules according to any one of aspects 20 to 24 for use in regenerative medicine.
27. Plasma enriched in platelets and plasma molecules for use according to aspect 26, wherein the plasma is used in the treatment of injured tissues.
28. Plasma enriched in platelets and plasma molecules according to any one of aspects 20 to 24 for use in the treatment of inflammatory diseases.
29. Plasma enriched in platelets and plasma molecules according to any one of aspects 20 to 24 for use in the treatment of rheumatic diseases.
30. Plasma enriched in platelets and plasma molecules for use according to any one of aspects 25 to 29, wherein the enriched plasma is administered to the same patient from which the anticoagulated blood sample of step i) was obtained.
31. Plasma enriched in platelets and plasma molecules for use according to any one of aspects 25 to 29, wherein the enriched plasma is administered to a different patient from which the anticoagulated blood sample of step i) was obtained.
32. Cosmetic use of the plasma according to any of aspects 20 to 24.
33. *In vitro* use of a hydrogel for concentrating blood or plasma, wherein the hydrogel is as defined in any one of aspects 1 and 5-12.
34. Use according to aspect 33, wherein the plasma is obtained from blood by removal of RBCs and optionally also removal of WBCs.

The invention will be described by means of the following examples which are to be considered merely illustrative and not limiting of the scope of the invention.

### EXAMPLES

### Hydrogel preparation process

The hydrogel used in the step ii) of the method of the invention to carry out the absorption of water from the plasma is composed of the monomer hydroxyethyl acrylamide (HEAA) (Sigma-Merck, ref. 697931). The polymerization of the HEAA was carried out by redox reaction, using N,N'-Methylenebisacrylamide (MBAAm) (Sigma-Merck, ref. 146072) as crosslinking agent and Ammonium Persulfate (APS) (Sigma-Merck, ref. 248614) and N'-Tetramethylethylenediamine (TEMED) (Sigma-Merck, ref. 411019) as initiators (See Table 1). The solvent employed was distilled water. Polymerization was carried out for 1h at room temperature.

**Table 1. Quantities used for the manufacture of HEAA gel in an example of ultraviolet light polymerization.**

| Reagents | Quantities per 500 ml of distilled water |
|---|---|
| Hydroxyethyl acrylamide (HEAA) | 148.876 g |
| Type II distilled water | 500 g |
| N,N'-Methylenebisacrylamide (MBAAm) | 3.723g |
| Ammonium Persulfate (APS) | 1.586 g |
| N'-Tetramethylethylenediamine (TEMED) | 3.073 g |

After polymerization, the hydrogel was washed with distilled water to remove any monomer residue that could have not polymerized, and the hydrogel was then dehydrated by applying a temperature of 56°C. Granulation of the dehydrated hydrogel block was carried out by mortar and pestle grinding, and then the desired particle size was selected by sieving.

### Description of the method of the invention

1. The patient's blood was extracted using tubes for 9mL extraction with anticoagulant, in this example sodium citrate, 3.8% as an anticoagulant. The volume of blood to be extracted was at least four times more than the desired final concentrated plasma.
2. The blood tubes were centrifuged at a speed of 1250g for 8 minutes to obtain a plasma whose concentration of platelets and proteins is similar to that of blood.
3. The entire plasma fraction was collected not including neither white blood cells nor red blood cells.
4. The plasma fraction obtained in step 3 was put in contact with the hydrogel (for example, 0.5 g of hydrogel per 4 mL of plasma for a hydrogel particle size between 0.5-1mm) for 5 minutes.
5. The hydrogel-plasma mixture was filtered by a filter with pore size greater than 5 µm so as not to retain proteins or platelets. This filtration was facilitated by centrifugation of 500xg for 2 minutes to obtain the final concentrated plasma.
6. The concentrated plasma obtained was activated by 10% CaCl₂ in proportion 20µl CaCl₂ per 1 mL plasma.

### Technical analysis of the product obtained

In order to verify that the concentrated plasma according to the invention (hereinafter nPRP) has properties in tissue regeneration, different experiments were conducted to see if an improvement in treatment is achieved compared to conventional PRPs. Specifically, a comparison has been made with:
- a standard PRP called sPRP, obtained by a centrifugation of 580xg for 8 minutes. After this centrifugation, the 2 mL of plasma above the leukocyte layer were collected. As in the PRP of the invention (nPRP), this plasma was subsequently activated with CaCl₂ and was the supernatant (serum) remaining after the formation of the clot that was used in functional and descriptive tests.
- a PRP obtained by centrifugation and subsequent water absorption according to the patent document WO 2006/086199 A1. This PRP was obtained by centrifugation of 1200 x g for 8 minutes. This PRP was then contacted with the hydrogel Bio-Gel P10 Media catalogue number: 150-4140 (acrylamide and N,N'-methylene-bis-acrylamide) described in application WO 2006/086199 A1. The contact between the hydrogel and PRP was maintained for 6 minutes and the proportion between them was 0.5 g of hydrogel per 4 mL of PRP. Then, the separation of hydrogel and PRP was carried out by filtration (100 µm nylon filters). This comparative PRP is herein referred to as WOPRP.

### nPRP versus sPRP comparative data

### Concentration of platelets, total proteins and albumin

The new PRP presented in this application (nPRP) is a platelet-rich plasma (PRP), with an average platelet concentration of 1.9 times (range 1.73 - 2.37) with respect to blood levels (Figure 1). If these results are compared with those obtained in a standard PRP (sPRP), similar levels of platelets is found, with no statistically significant differences between the two systems.

The levels of total proteins and albumin have been measured, this being indicative of the concentration of the biomolecules present in the extra-platelet medium. Figure 2 shows the ratio of total protein concentration and albumin in both sPRP and nPRP with respect to baseline blood levels. The results show a statistically significant increase in protein levels, and also albumin specifically, in the new formulation compared to the standard.

### Concentration of growth factors (ELISAs)

With the analysis of total proteins it has been possible to verify that it is possible to double the proteins present in the plasma. To verify the concentration of some of the proteins of interest specifically, the analysis of different growth factors has been carried out using the ELISA technique. The authors of the present invention wanted to verify that platelet factors were concentrated with standard PRP, due to platelet enrichment. To this end, the platelet factors PDGF-AB, VEGF and TGF-B were measured, and these were similar in both formulations, increasing proportionally to platelet concentration.

After this confirmation, two plasma factors (IGF-I and HGF) were measured to confirm that this new processing is capable of concentrating these together with platelets, unlike conventional PRPs, where only platelet factors are concentrated.

Figure 3 shows the relationship of the concentration of plasma growth factors in the different systems, nPRP and sPRP, relative to baseline levels. Both HGF factor and IGF-I were approximately doubly enriched in the new formulation compared to the standard one.

### Cell viability

These results confirm that sPRP and nPRP are two products with different composition, which could potentially generate differences in their bioactivity, for which an *in vitro* study was carried out where their impact on cell proliferation was measured.

In order to analyze whether nPRP has a greater response in cell proliferation compared to conventional formulas, cellular media were supplemented with 10% of serum from different formulations, after activation with CaCl₂. Figure 4 shows the growth rate of dermal fibroblasts grown *in vitro* with media supplemented with sPRP and nPRP of 5 patients. Measurements were made at 96h. For this, a method of measuring cell viability based on luminescence was used. As shown in Figure 4, the nPRPs of the 5 patients promote greater cell growth on dermal fibroblasts than the standard formula.

### nPRP versus WOPRP comparative data

### Concentration of platelets, total proteins

The new PRP presented in this application, nPRP, is a platelet-rich plasma (PRP), with a statistically significant average platelet concentration superior to that of WOPRP (Figure 5).

The levels of total proteins have been measured, this being indicative of the concentration of the biomolecules present in the extra-platelet medium. Figure 6 shows the ratio of total protein concentration in both WOPRP and nPRP with respect to baseline levels. The results show a total protein concentration ratio in the nPRP of 2.28 (range 2.07 - 2.61), being significantly higher than the WOPRP range.

### Concentration of growth factors (ELISAs)

With the analysis of total proteins it has been possible to verify that it is possible to concentrate at least twice the proteins present in the plasma. To verify the concentration of some of the proteins of interest specifically, the analysis of different growth factors has been carried out using the ELISA technique. The authors of the present invention wanted to verify that platelet factors were concentrated with WOPRP, due to platelet enrichment. To this end, the platelet factors PDGF-AB and VEGF were measured (Figure 7). In the case of PDGF, nPRP showed a higher ratio of molecular concentration to WOPRP.

### Cell viability

These results confirm that nPRP and WOPRP are two products with different composition, which could generate differences in their bioactivity, for which an *in vitro* study was conducted where their impact on cell proliferation was measured.

In order to analyze whether nPRP has a greater response in cell proliferation with respect to the WOPRP formula, the cellular media were supplemented with 10% of the serum from the different formulations, after its activation with CaCl₂. Figure 8 shows the growth values of dermal fibroblasts cultured *in vitro* with media supplemented with nPRP and WOPRP of 9 patients. Measurements were made at 96h. For this, a method of measuring cell viability based on luminescence was used. As shown in Figure 9, nPRP promotes greater cell growth on dermal fibroblasts than the WOPRP formula, demonstrating that changes in nPRP composition lead to a better biological response.

## Claims

1. An *in vitro* method for producing a plasma enriched in platelets and plasma molecules comprising:
(i) obtaining a red blood cell-free plasma from an anticoagulated blood sample of a subject, wherein the plasma comprises platelets and plasma molecules; and
(ii) concentrating the platelets and plasma molecules comprised in the plasma obtained in step i) by contacting said plasma with a hydrogel, wherein the hydrogel comprises a polymer network, and the polymer network comprises monofunctionalized N-hydroxyalkyl acrylamide monomeric units; or N-hydroxyalkyl alkylacrylamide monomeric units; or hydroxyalkyl acrylate monomeric units; or hydroxyalkyl alkylacrylate monomeric units; and is cross-linked.

2. Method according to claim 1, wherein in step i) the plasma obtained from the anticoagulated blood sample of a subject is free of red blood cells and white blood cells.

3. Method according to any one of claims 1 or 2, wherein the red blood cell free plasma of step i) is obtained by centrifugation at a speed of between 800 to 1600 g for a time of 5 to 20 minutes from the anticoagulated blood sample, and isolating the supernatant and buffy coat layers from the centrifuged blood sample.

4. Method according to claim 2, wherein the red blood cell free and white blood cell free plasma of step i) is obtained by centrifugation at a speed of between 800 and 1600 g for a time of 5 to 20 minutes from the anticoagulated blood sample, and isolating the supernatant layer from the centrifuged blood sample.

5. Method according to any one of claims 1 to 4, wherein the polymer network comprised in the hydrogel used in step (ii) comprises N-hydroxyethyl acrylamide (HEAA) monomeric units.

6. Method according to any one of claims 1 to 5, wherein the polymer network comprised in the hydrogel used in step (ii) comprises bifunctionalized acrylic monomeric units, preferably N,N-Methylenebis(acrylamide) (MBAA) monomeric units.

7. Method according to any one of claims 1 to 6, wherein the polymer network comprised in the hydrogel used in step (ii) comprises the monofunctionalized monomeric units in an amount of at least 60% by weight with respect to the weight of the polymer network.

8. Method according to any one of claims 1 to 7, wherein the method further comprises an additional step comprising separating the concentrated plasma from the hydrogel-plasma mixture.

9. Method according to any one of claims 1 to 8, further comprising the step of activating the platelets in the concentrated plasma.

10. Method according to claim 9, wherein the activation of platelets is performed by the addition of a compound selected from calcium chloride, thrombin and collagen, preferably calcium chloride.

11. Method according to any one of claims 1 to 10, wherein the platelet and total protein concentration of the concentrated plasma is at least 1.5 times higher than that of the anticoagulated blood sample of step i).

12. Method according to any one of claims 1 to 11, wherein the concentration of IGF-I is at least 1.5 times higher than that of the anticoagulated blood sample of step i) and/or the concentration of HGF is at least 1.5 times higher than that of the anticoagulated blood sample of step i).

13. Plasma enriched in platelets and plasma molecules obtained by the method according to any one of claims 1 to 12.

14. Plasma according to claim 13 for use in regenerative medicine or in the treatment of inflammatory diseases.

15. *In vitro* use of a hydrogel for concentrating blood or plasma, wherein the hydrogel comprises a polymer network, and the polymer network comprises monofunctionalized N-hydroxyalkyl acrylamide monomeric units; or N-hydroxyalkyl alkylacrylamide monomeric units; or hydroxyalkyl acrylate monomeric units; or hydroxyalkyl alkylacrylate monomeric units; and is cross-linked.
